Europäisches Patentamt

European Patent Office

Office européen des brevets

(11) Publication number : **0 631 222 A1**

## (12) EUROPEAN PATENT APPLICATION

(21) Application number : **94303837.2**

(22) Date of filing : **26.05.94**

(51) Int. Cl.⁵ : **G06F 3/00**

(30) Priority : **21.06.93 JP 148995/93**

(43) Date of publication of application :
**28.12.94 Bulletin 94/52**

(84) Designated Contracting States :
**DE FR GB**

(71) Applicant : **INTERNATIONAL BUSINESS MACHINES CORPORATION**
**Old Orchard Road**
**Armonk, N.Y. 10504 (US)**

(72) Inventor : **Misono, Shinji**
**201 3-10-5, Kamikitazawa,**
**Setagaya-ku**
**Tokyo (JP)**
Inventor : **Hama, Toshiyuki**
**105 2-34-5, Komazawa,**
**Setagaya-ku**
**Tokyo (JP)**
Inventor : **Etoh, Hiroaki**
**206 1-8-23, Inukura,**
**Miyamae-ku**
**Kawasaki-shi, Kanagawa-ken (JP)**

(74) Representative : **Williams, Julian David**
**IBM United Kingdom Limited,**
**Intellectual Property Department,**
**Hursley Park**
**Winchester, Hampshire SO21 2JN (GB)**

(54) **Gazing point estimation device.**

(57)   The device described enables calibration data indispensable for calculation of a gazing point to be fetched and also an estimation of a gazing point to be determined by calculating a pupil center position through image-processing data obtained via a camera, increasing the brightness of a gazing point on a display screen according to the calculated values and calibration data, measuring the change in pupil area at the moment, establishing a correlation, thereby improving the operability of a gazing point estimation system.

FIG. 1

This invention relates to a system for interfacing a user with a computer, and more particularly to a system that estimates a user's gazing point on a screen.

One type of interface between a computer and a user who is unable to use his/her hands due to, for example, disability, utilises the user's gaze. Of interfaces of this kind, the least burdensome to users and highest in the degree of freedom is a non-contact system that estimates a user's gazing point on the screen of a display using a video camera and a source of infrared radiation. Examples of known non-contact man-machine interfaces are described in Richard A. Bolt, "Evolutionary Theory of Man-Machine Interfaces," Personal Media (1986), D. L. Gardener, Looking at Looking", in Proceedings of the PS/2 Future Symposium, A Vision of the Future, The Ideal 1993 Personal System, Boca Raton, FL. April 1988.

In such a device, data obtained through a video camera is first image-processed to determine the contour of the user's eye and the central position of his/her pupil, and then reflected light from the cornea is measured by casting infrared rays on the user's eye. A gazing point on the screen is estimated by using the data thus obtained.

However, this conventional device poses problems. One is a problem of calibration. Estimation of a gazing point entails obtaining data as to reference points (such as the four corners of the screen) in order to specify the absolute position of a gazing point. Such data is needed at the beginning of work and also each time the user's posture changes greatly. Moreover, inasmuch as the correlative relation between the movement of the eye and a change from one gazing point to another includes parameters dependent on each individual, calculation data becomes necessary for determination of such parameters. Hence, the conventional system requires reference point data and calculation data to be fetched each time there is a change from one user to another. Such data is usually collected by a user's gazing at two or more predetermined points in sequence on the screen prior to beginning work. However, this method is disadvantageous in that a user's flow of thought is interrupted because the object to be gazed at has nothing to do with the work the user wishes to do at the outset.

Another problem is as to how to determine the results of gazing point estimation. In this respect, there is no effective method for the system to judge whether or not a spot on the screen estimated from the above data exactly coincides with the position of the user's gazing point. For this reason, it has conventionally been common to employ a method of determination aided by a deterministic signal, such as a blink, sent from the user. In this connection, a proposal to provide a button exclusively used for sending a deterministic signal has also been presented, but this proposed method cannot be applied to users who cannot use their hands. See P. J. Kennedy, "Point of Regard Tracking Device," IBM TDB n10A 03-92 pp488-490, for example.

In accordance with the present invention there is now provided a gazing point estimation device comprising: a display; means for calculating a central position of a user's pupil gazing on the screen of the display; means for detecting changes in the area of the user's pupil; means for estimating the user's gazing point on the screen based on calculation data obtained by the calculating means and calibration data; and means for changing the brightness of the estimated gazing point; wherein a current pupil center position and an estimated gazing point are accumulated in the estimating means to serve as calibration data for estimating the user's gazing point in response to a change in the area of the user's pupil being detected by the detecting means and no change in pupil center position being detected by the calculating means after a change in brightness.

Viewing the present invention from another aspect, there is now provided a method of estimating gazing point on a display screen, the method including: a) calculating a central position of a user's pupil gazing on the display screen; b) detecting changes in the area of the user's pupil; c) estimating the user's gazing point on the screen based on calculation data obtained in step a) and calibration data; d) changing the brightness of the estimated gazing point; and e) accumulating a current pupil center position and an estimated gazing point to serve as calibration data for estimating the user's gazing point in response to a change in the area of the user's pupil being detected and no change in pupil center position being detected after a change in brightness.

Viewing the present invention from yet another aspect, there is now provided an interface system comprising: a display; means for calculating the central position of a user's pupil gazing on the screen of said display; means for detecting changes in the area of said user's pupil; means for estimating said user's gazing point on the screen based on calculation data obtained by said pupil center position calculating means and calibration data; means for changing the brightness of said estimated gazing point; means for establishing a man-machine interface through accumulating a current pupil center position and an estimated gazing point in said estimating means to serve as calibration data for estimating said user's gaze and estimating a gazing point on the screen by said estimating means in response to a change in the area of said user's pupil being detected by said pupil area change detecting means and no change in pupil center position being detected by said pupil center position calculating means after said change in brightness.

The present invention thus improves the operability of a gazing point estimation device by obviating the need to do special calibration work and thus enabling the reference point data and calibration data required for estimation of a gazing point on a display screen to be fetched without interrupting the user's flow of thought and also enabling results of estimation to be reliably determined.

The present invention enables calibration data indispensable for estimation of a gazing point to be fetched and an estimation of a gazing point to be determined by increasing the brightness of a gazing point estimated on a display screen, measuring the change in pupil area at that moment, establishing a correlation, and thus improves on the conventional estimation device based on image processing or infrared ray photometry.

A preferred embodiment of the present invention will now be described, by way of example only, with reference to the accompanying drawings, in which:

Figure 1 is a block diagram gazing point estimation system of the present invention;

Figure 2 shows a pupil center position coordinate system;

Figure 3 shows a screen coordinate system representing an estimated gazing point;

Figure 4 is a flowchart of the estimation of a gazing point of this invention;

Figure 5 shows an example of a pointer (shaded section); and

Figure 6 shows an example of a pointer (shaded section).

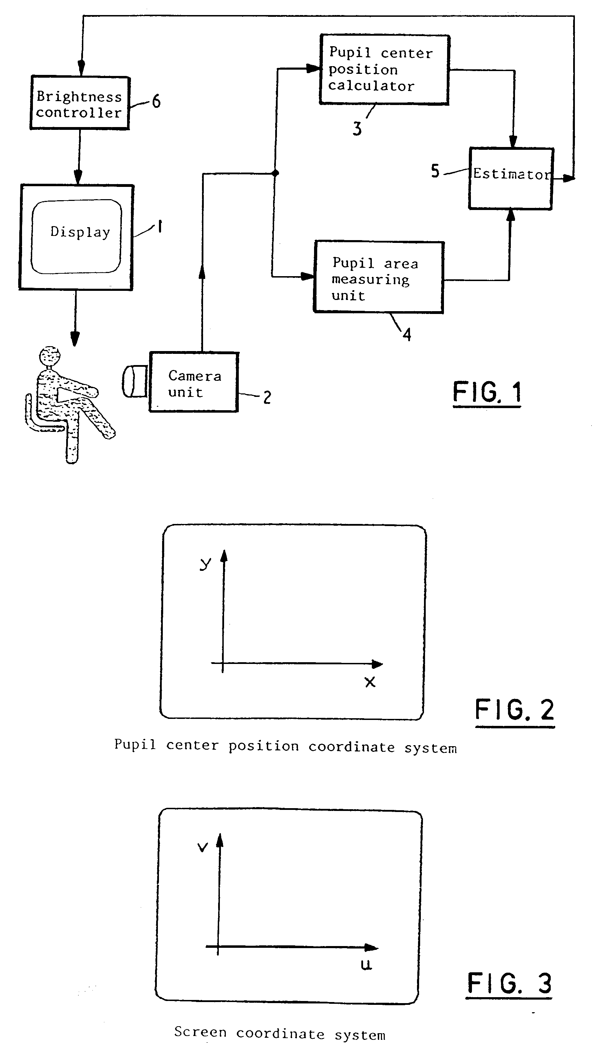

Figure 1 shows a gazing point estimation device of the present invention. The device includes a display 1, on the screen of which characters are displayed. There is also a camera unit 2, which consists of a video camera, a source of infrared radiation, and a measuring instrument if necessary, whereby video data of an image of the eye is collected through the video camera. There is also a pupil center position calculator 3, which calculates the central position of a user's pupil by performing image processing on the video data from the camera unit 2, and in some devices further processes data as to light reflected in the cornea from an infrared radiation source so as to calculate the central position of a user's pupil even more accurately. There is also a pupil area measuring unit 4, which calculates the area of the pupil by performing image processing on video data from the camera unit 2. The present invention does not require any absolute area values. Calculating the relative temporal changes alone is sufficient. Since a detailed explanation of these constituent devices has already been given in the above-mentioned references, explanation of this embodiment shall omit any further detailed explanation here. There is also an estimator 5, which estimates a gazing point on the screen from the pupil center position thus calculated and its calibration data. (Such a gazing point thus estimated is hereinafter called an estimated gazing point.) The estimator 5 then accumulates calibration data necessary for the subsequent gaze estimation from data obtained according to the calculated pupil center position and its change in brightness. The brightness controller 6 varies the brightness of an estimated gazing point on the screen. It will be appreciated that the pupil center position calculator 3, the estimator 5, the brightness controller 6, and the pupil area measuring unit may be implemented by hard-wired logic or by a computer system such as a personal computer configured by computer program code.

The following describes a practical method for estimating a gazing point on the screen from a pupil center position obtained by image processing. It will be appreciated that such a method may be implemented by hard-wired logic or by computer program code for controlling a computer system.

Provided that a pupil center position is represented by a (x, y) coordinate system, as in Figure 2, and an estimated gazing point, or a point on the screen, is represented by a (u, v) coordinate system, as in Figure 3, the problem here is to calculate an estimated gazing point (u, v) from a pupil center position (x, y).

Suppose that the functional relationship between coordinates (x, y) and coordinates (u, v) has been determined. The following equation including parameters a, b, c, and d is considered as the simplest function:

$$\begin{cases} u = ax + b \\ v = cy + d \end{cases} \qquad (1)$$

To estimate a gazing point from this function, it is necessary to determine the parameter values of this function. The required data for this parameter value determination is calibration data. Letting the minimum required number of data elements be $N_p$, we obtain $N_p = 2$ from the estimation function (1), for instance, because coordinates (a, b, c, d) can be uniquely determined if the pupil center position data is obtained at two locations.

Moreover, it is presupposed that the user or system has determined in advance a critical value, c, which denotes the discrepancy (distance) between an estimated gazing point (u, v) and a real gazing point $(u_r, v_r)$:

$$|(u, v) - (u_r, v_r)| < c \qquad (2)$$

If this equation is satisfied, the estimation shall be regarded as correct. (Here, "$|\ |$" is the distance between 2 points, or in other words, the value of the following equation.)

$$|(u,v) - (u_r, v_r)| = \sqrt{(u - u_r)^2 + (v - v_r)^2}$$

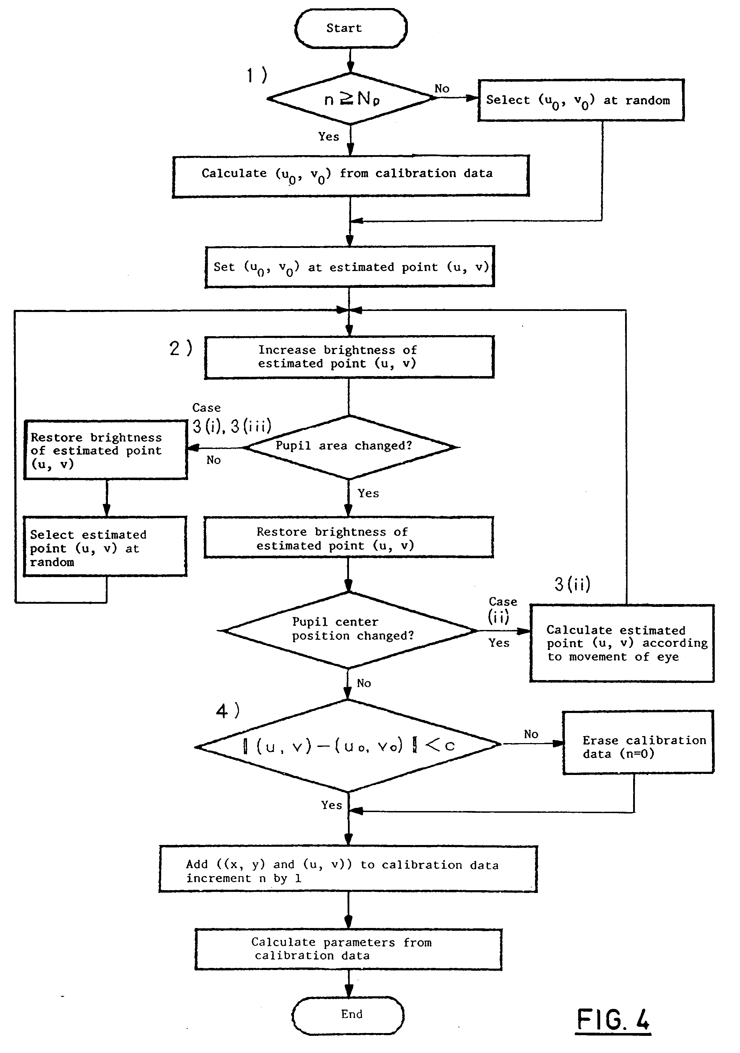

Provided that an estimation function, a required number of data elements (Np), and a critical value (c) have been determined in advance, the flowchart shown in Figure 4 is obtained, which illustrates a method of dynamically accumulating and updating the calibration data required for determination of the parameters of the estimation function.

The following describes each part of the flowchart shown in Figure 4.

1) Before estimation is started, the number, n, of existing calibration data elements is compared with the required number of data elements, Np. If $n < Np$, then an estimated gazing point $(u_0, v_0)$ is selected at random, because the data requirement for the estimation is not yet satisfied. If $n \geqq Np$, then an initial estimated gazing point $(u_0, v_0)$ is calculated by using a predetermined estimation function.

2) The initial estimated gazing point $(u_0, v_0)$ thus obtained is used as an estimated gazing point $(u, v)$, the brightness of this spot being increased to check for changes in pupil center position and pupil area. If the estimated gazing point approximates the actual gazing point $(u_r, v_r)$, the pupil center position will not change and the pupil area will decrease. By contrast, if the estimated gazing point is apart from the real gazing point, one of the following three situations will arise:

(i) Neither the pupil area nor pupil center position changes. This arises when the user is fixing his eye on the current gazing point.

(ii) The pupil center position changes and then the pupil area changes. This arises when the user moves his eye toward a brighter estimated gazing point.

(iii) The pupil center position changes, but the pupil area does not change. This arises when the user casts his eye on a location having no relationship to the estimated gazing point in question.

In these three situations (i), (ii), and (iii), estimated gazing points are successively updated.

3) Updating estimated gazing points

3-(i) and 3-(iii): This processing applies to the above occasions (i) and (iii). On both occasions, another estimated gazing point $(u, v)$ is selected at random because the only given information is that the current estimated gazing point is incorrect.

3-(ii): On this occasion, a new estimated point is calculated according to eye movement information. For instance, when the eye has moved in the upper-right direction, the new estimated point is set at the lower-left of the current estimated point.

4) A check is made to see whether or not the distance between the final estimated point $(u, v)$ and the initial estimated point $(u_0, v_0)$ falls within the critical value, c. If it falls within the critical value, c, the current data $((x, y,)$ and $(u, v))$ is added to the existing estimated data. If the distance is not less than the critical value, c, it is because the existing estimated data is no longer valid for some reason or other, e.g., a change from one user to another or a change in the posture of the same user. The existing data is then erased, the number of calibration data elements is reset to 0, and the current data is registered as the first calibration data element.

The present invention estimates a gazing point by repeating the above operations thereby allowing wider applications as a man-machine interface, including printing, vocalizing, enlarging, and illustrating a gazed zone.

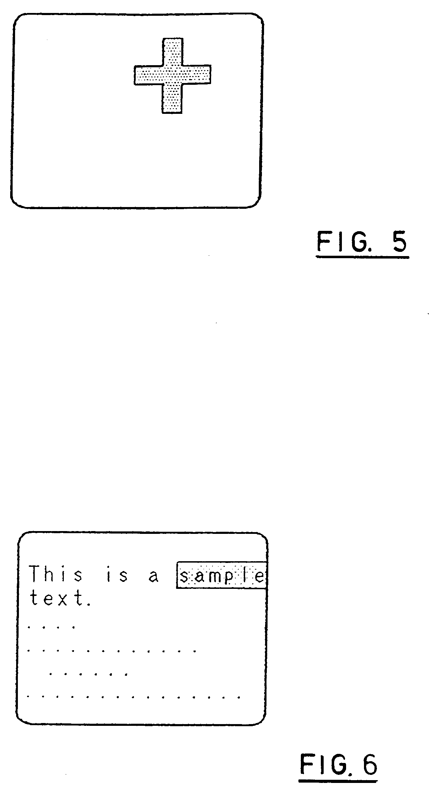

Although in the above embodiment, the explanation has been made so far in supposition of such special-shape pointers as shown in Figure 5, the pointers may be of any shape. In this respect, it is also possible to use display data itself instead of pointers, as shown in Figure 6. Again, this invention can be worked even on a device whose camera unit consists of a video camera alone without using infrared rays if only the data required for determination of a pupil center position and the area of the pupil is obtained.

From the foregoing it will be appreciated that the invention obviates the need for special calibration work, enables the calibration data required for estimation of a gazing point to be fetched without interrupting a user's flow of thought and the results of estimation to be determined exactly as well, thus helping to improve the operability of a gazing point estimation device.

## Claims

1. A gazing point estimation device comprising:
   a display (1);
   means (3) for calculating a central position of a user's pupil gazing on the screen of the display (1);
   means (4) for detecting changes in the area of the user's pupil;
   means (5) for estimating the user's gazing point on the screen based on calculation data obtained by the calculating means (3) and calibration data; and
   means (6) for changing the brightness of the estimated gazing point; wherein a current pupil center position and an estimated gazing point are accumulated in the estimating means (5) to serve as calibration data for estimating the user's gazing point in response to a change in the area of the user's pupil being detected by the detecting means (4) and no change in pupil center position being detected by the calculating means (3) after a change in brightness.

2. A device as claimed in Claim 1, wherein the estimating means further estimates another gazing point when said change in pupil area and a change in pupil center position are detected.

3. A device as claimed in Claim 1, wherein the brightness changing means moves a pointer provided as a mouse cursor or a editing cursor on the screen to an estimated gazing point and increases the brightness of the pointer zone only.

4. A device as claimed in Claim 1, wherein the brightness changing means increases the brightness of the display data in the position of an estimated gazing point on the screen.

5. A computer system comprising a device as claimed in any preceding claim.

6. A method of estimating gazing point on a display screen, the method including:
   a) calculating a central position of a user's pupil gazing on the display screen;
   b) detecting changes in the area of the user's pupil;
   c) estimating the user's gazing point on the screen based on calculation data obtained in step a) and calibration data;
   d) changing the brightness of the estimated gazing point; and
   e) accumulating a current pupil center position and an estimated gazing point to serve as calibration data for estimating the user's gazing point in response to a change in the area of the user's pupil being detected and no change in pupil center position being detected after a change in brightness.

**FIG. 1**

**FIG. 2**

Pupil center position coordinate system

**FIG. 3**

Screen coordinate system

```
                          ┌──────────┐
                          │  Start   │
                          └────┬─────┘
                               │
        1)              ◇─────────────◇    No    ┌─────────────────────────┐
                       ◇   n ≧ Nₚ     ◇─────────▶│ Select (u₀, v₀) at random│
                        ◇───────────◇            └───────────┬─────────────┘
                            │ Yes                            │
              ┌─────────────▼──────────────────────┐         │
              │ Calculate (u₀, v₀) from calibration │         │
              │ data                                │         │
              └─────────────┬──────────────────────┘         │
                            │◀──────────────────────────────┘
              ┌─────────────▼──────────────────────┐
              │ Set (u₀, v₀) at estimated point (u, v)│
              └─────────────┬──────────────────────┘
```

1) $n \geq N_p$ — No → Select $(u_0, v_0)$ at random

Yes → Calculate $(u_0, v_0)$ from calibration data

Set $(u_0, v_0)$ at estimated point $(u, v)$

2) Increase brightness of estimated point $(u, v)$

Case 3(i), 3(iii) — Pupil area changed?

No → Restore brightness of estimated point $(u, v)$ → Select estimated point $(u, v)$ at random

Yes → Restore brightness of estimated point $(u, v)$

Pupil center position changed?

Case (ii) Yes → 3(ii) Calculate estimated point $(u, v)$ according to movement of eye

No →

4) $\| (u, v) - (u_0, v_0) \| < c$

No → Erase calibration data (n=0)

Yes → Add $((x, y)$ and $(u, v))$ to calibration data increment n by 1

Calculate parameters from calibration data

End

<u>FIG. 4</u>

7

FIG. 5

FIG. 6

EP 0 631 222 A1

| | European Patent Office | EUROPEAN SEARCH REPORT | Application Number EP 94 30 3837 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | US-A-5 204 703 (HUTCHINSON T. E.) <br> * column 4, line 19 - column 6, line 38 * <br> * column 17, line 20 - column 20, line 10 * <br> --- | 1,5,6 | G06F3/00 |
| A | US-A-5 036 347 (TSUNEKAWA T.) <br> * column 3, line 32 - line 46 * <br> * abstract; claims 1,4 * <br> --- | 1,6 | |
| A | GB-A-1 581 018 (STANDARD TELEPHONES AND CABLES) <br> * the whole document * <br> --- | 1,2,5,6 | |
| A | ICL TECHNICAL JOURNAL <br> vol. 7, no. 2 , November 1990 , HITCHIN GB <br> pages 384 - 411 XP176706 <br> EPWORTH R. 'Eye Movements for a Bidirectional Human Interface' <br> * page 401, line 3 - line 13 * <br> --- | 1,3,4 | |
| A | EP-A-0 055 338 (IBM) <br> * page 17, line 15 - page 18, line 34 * <br> ----- | 1,6 | TECHNICAL FIELDS SEARCHED (Int.Cl.5) <br><br> G06F <br> A61B |

The present search report has been drawn up for all claims

| Place of search | Date of completion of the search | Examiner |
|---|---|---|
| THE HAGUE | 29 September 1994 | Bailas, A |